# EUROPEAN PATENT SPECIFICATION

(11) **EP 3 148 620 B1**
(45) Date of publication and mention of the grant of the patent: **23.04.2025**
(21) Application number: 15724671.1
(22) Date of filing: 26.05.2015
(51) Int. Cl.: A61M 5/31

(54) **METHOD FOR STORING AN EMULSION-ADJUVANTED VACCINE IN A LUBRICATED MEDICAL INJECTION DEVICE**
VERFAHREN ZUR AUFBEWAHRUNG EINES EMULSIONSUNTERSTÜTZTEN IMPFSTOFFS IN EINER GESCHMIERTEN MEDIZINISCHEN INJEKTIONSVORRICHTUNG
PROCÉDÉ PERMETTANT DE STOCKER UN VACCIN AVEC ADJUVANT ÉMULSIONNÉ DANS UN DISPOSITIF D'INJECTION MÉDICAL LUBRIFIÉ

(30) Priority: 26.05.2014 EP 14305781
(43) Date of publication of application: 05.04.2017
(73) Proprietor: Becton Dickinson France, 38800 Le Pont-de-Claix (FR)
(72) Inventor: HAMEL, Jean-Bernard, F-38500 Saint Cassien (FR); BONHOURE, Fabien, 1278 LA RIPPE (CH); JOUFFRAY, Sébastien, F-38410 Saint Martin d'Uriage (FR); BRALET, Nicolas, F-38800 Champagnier (FR)
(74) Representative: Regimbeau
(86) International application number: PCT/EP2015/061594
(87) International publication number: WO 2015/181173

(56) References cited:
- EP-A2- 0 201 915
- WO-A1-2011/092536
- WO-A1-2013/178647
- WO-A2-2009/053947
- WO-A2-2013/045571
- US-A- 5 338 312
- US-A1- 2011 313 363
- VIOLA SCHULTZE ET AL: "Safety of MF59(TM) adjuvant", VACCINE, vol. 26, no. 26, 1 June 2008 (2008-06-01), AMSTERDAM, NL, pages 3209 - 3222, XP055536349, ISSN: 0264-410X, DOI: 10.1016/j.vaccine.2008.03.093
- VOGEL FREDERICK R ET AL: "Emulsion-based adjuvants for influenza vaccines", EXPERT REVIEW OF VACCINES, FUTURE DRUGS, LONDON, GB, vol. 8, no. 4, 1 April 2009 (2009-04-01), pages 483 - 492, XP009149827, ISSN: 1476-0584

## Description

### FIELD OF THE INVENTION

The invention relates to a method for storing an emulsion-adjuvanted vaccine in a lubricated medical injection device.

### TECHNICAL BACKGROUND

Vaccines are today widely used to prevent number of diseases such as tuberculosis, poliomyelitis or smallpox and systematic vaccination campaigns have been successfully conducted to eradicate severe diseases in many areas. They usually comprise antigenic material intended to stimulate the immune system, together with adjuvants intended to boost the immune response. Such vaccines are commonly injected under a liquid form with injection devices.

Such injection devices such as syringes, pens or auto-injectors usually comprise a container intended to receive the vaccine to be injected and a plunger rod intended to move the stopper within the container so as to expel the vaccine therefrom at the time of injection.

A vaccine can either be stored in a vial and transferred into an injection device by the time of injection, or preferably stored into the injection device, ready to be used.

So-called pre-filled injection devices are now widespread because they present several advantages, such as preventing contamination of the vaccine during the transfer from the vial and reducing human errors on the volume or the type of vaccine to inject to the patient. Furthermore, pre-filled injection devices are also useful to simplify supply chain by providing a single package, which is particularly valuable during remote immunization campaigns.

However, vaccines are made from biological material and are sensitive towards their environment. They can be altered by the storage conditions, in particular temperature, but also by the container itself through interactions with its components. There is therefore a need to find an appropriate container able to maintain the integrity of the vaccines when they are stored for a long time in pre-filled injections devices. Furthermore, lubricant coatings, in particular made of silicone, are often applied on the inner surface of the container and/or on the surface of the stopper in order to provide lubrication of the injection device and thereby decrease the gliding force to move the stopper within the container by the time of injection.

Moreover, some vaccine formulations, which are called here "sensitive vaccine formulations" may present some interactions with the silicone of the pre-filled injection device. In particular, such interactions may happen with adjuvants that are present in the pharmaceutical composition to boost the immune response resulting from the injection and thereby enhance the potency of the vaccine itself.

Aluminum has been widely used as an adjuvant for this purpose but the concentration allowed in vaccine is now strongly limited by Chapter 21 of the US Code of Federal Regulations [610.15(a)]. With reduced concentration, some aluminum-based adjuvants may not be suitable or sufficiently potent to allow the development of new generation vaccines.

Therefore, as an alternative, a new class of adjuvants based on emulsions has been developed to stimulate a more effective immune response [1].

Such emulsion adjuvants can be classified into either water-in-oil or oil-in-water emulsions. Several emulsion adjuvants currently used for new generation vaccines contain squalene as the oil phase in an oil-in-water emulsion together with non-ionic surfactants as emulsifiers, in order to stabilize the emulsion [1].

Nevertheless, the applicant found that such emulsions could lead to a strong interaction with the silicone coating of a pre-filled injection device. In particular, the silicone could migrate into the oil phase of the emulsion, leading to a modification of the emulsion particle size and of the composition of this phase. The vaccine antigen itself could also be denatured and loose its biological function. Furthermore, injecting such modified composition to population would be inacceptable as this may lead to important side-effects, inappropriate immune response and non-effective immunization.

Conversely, the interaction between the emulsion and the silicone coating may deteriorate the functionality of the lubricant coating, especially its gliding properties. Indeed, the removed silicone from the lubricant coating could lead to non-lubricated areas on the inner surface of the injecting device that result in a difficult injection movement and to an unacceptable risk of injuries during the injection time.

As a result, there is a strong need to have an injection device with a lubricant coating showing no or negligible interaction with emulsion based vaccine adjuvants, while maintaining very good gliding capability for a safe and easy injection movement.

Furthermore, the integrity of the vaccine, the integrity of the coating and the gliding properties of the medical injection device need to be maintained over time i.e. to be stable for at least the shelf life of the injection device.

Document WO 2013/045571 teaches the use of a plasma treated silicone oil as a coating for reducing the number particles released in a pharmaceutical composition in contact with the coating. However, such particles are not a concern for emulsion-based vaccines.

### BRIEF DESCRIPTION OF THE INVENTION

A goal of the invention is thus to improve the compatibility between a lubricant coating and an emulsion-adjuvanted vaccine stored in a medical container comprising said lubricant coating.
In particular, the integrity and the gliding performances provided by the lubricant coating should be maintained even after an extended storage in contact with an emulsion-adjuvanted vaccine. Activation force and gliding force should be maintained as low as possible, to offer a safe and comfortable injection, even after an extended storage time of the medical container with a vaccine composition, The chemical nature and the emulsion population profile should also be preserved during the extended storage, in order to be injected in the human body in its most efficient form.

By "emulsion-adjuvanted vaccine" is meant in the present text a vaccine that contains an emulsion-based adjuvant. In addition, the distal end of a component or apparatus must be understood as meaning the end furthest from the hand of the user and the proximal end must be understood as meaning the end closest to the hand of the user, with reference to the injection device intended to be used with said component or apparatus. As such, in this application, the distal direction must be understood as the direction of injection with reference to the injection device, and the proximal direction is the opposite direction, i.e. the direction of the transfer of the product from the vial to the injection device.

The invention is as defined in independent claims 1, 13 and 14. Preferred embodiments are as defined in dependent claims 2-12.

According to an embodiment, the invention provides a method for storing an emulsion-adjuvanted vaccine in a medical container comprising a lubricant coating, said method comprising:
(a) providing a medical injection device comprising a barrel having an inner surface;
(b) forming a silicone oil layer on at least a part of the inner surface of the barrel that is intended to be in contact with the vaccine,
(c) carrying out an oxidizing plasma treatment of said layer so as to crosslink at least part of the silicone oil layer and form a lubricant coating,
(d) filling the barrel with the emulsion-adjuvanted vaccine.

This method allows maintaining the stability of the emulsion-adjuvanted vaccine overtime while preserving the integrity of the lubricant coating during storage and transportation. At the time of injection, a potent vaccine can thus be administered to people with a high level of safety and comfort for the medical staff.

The emulsion of the emulsion-based adjuvant is advantageously an oil-in-water emulsion and the oil phase may advantageously comprise squalene.

According to an advantageous embodiment of the invention the barrel of the medical injection device is made of glass.

According to an advantageous embodiment of the invention, the silicone oil has a viscosity between 900 and 1200 centiStokes at 25°C before the plasma treatment.

Advantageously, the lubricant coating has a thickness between 90 and 400 nm, measured by reflectometry.

Advantageously, the medical injection device comprises a syringe having an internal volume of 1 ml.

According to an advantageous embodiment of the invention, the silicone oil layer has a weight between 0.1 and 0.4 mg.

In a further advantageous embodiment, the storing method of the present invention may comprise providing a stopper in gliding engagement with the inner surface of the barrel, and forming a silicone oil layer on at least a part of said stopper that is intended to be in contact with the vaccine and exposing said silicone oil layer to an oxidizing plasma treatment so as to form a lubricant coating on said part of the stopper. A stopper lubricated according to this embodiment also contributes to preserve the stability of the emulsion-based adjuvant and very good gliding performances.

According to other advantageous embodiments that may be considered independently or in combination:
- at least 90% of the inner surface of the barrel is coated with said lubricant coating;
- the oxidizing plasma treatment is carried out in an atmosphere comprising oxygen and argon;
- the atmosphere of said oxidizing plasma contains oxygen and argon with respective partial pressures comprised between 15 and 30% for oxygen and between 85 and 70% for argon;
- the exposure of the silicone oil layer to the plasma is performed during between 10 and 40 seconds;
- the oxidizing plasma is generated by radio-frequency, with a power ranging from 50 to 300W, and under a vacuum in the range 1.33-13.3 Pa (10-100 mTorr) in absolute value.

Another aspect of the present invention is a medical injection device comprising a lubricant coating and containing an emulsion-adjuvanted vaccine in contact with said lubricant coating, said medical injection device being directly obtained by the previously described storing method. This injection device is particularly valuable for its low level of interaction between the lubricant coating and the emulsion-adjuvanted vaccine. Consequently, the emulsion profile and composition are not altered and the potency of the vaccine is maintained up to the time of injection. The lubricant coating integrity and gliding performances are also preserved and permit a safe and smooth injection after extensive storage and transportation.

### BRIEF DESCRIPTION OF THE DRAWINGS

Other features and advantages of the invention will become apparent from the detailed description to follow, with reference to the appended drawings, in which:

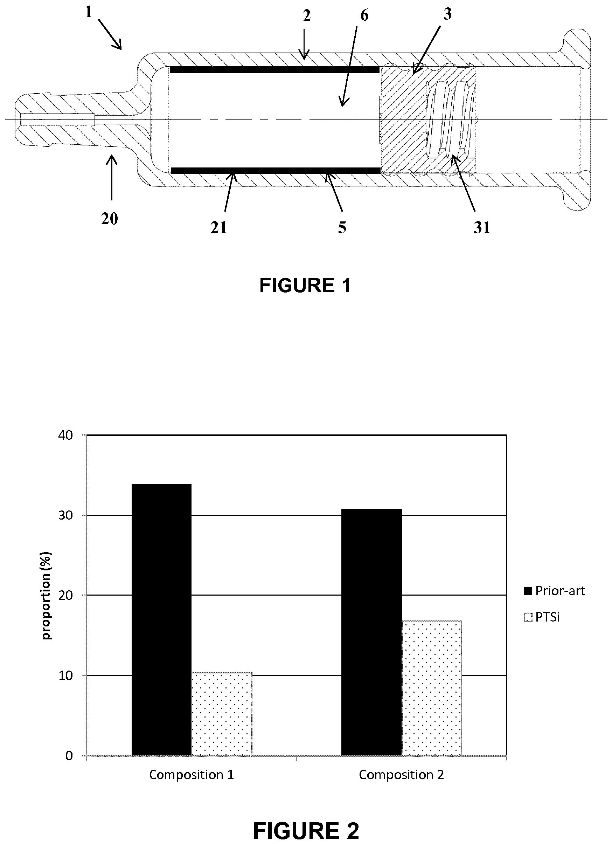

- Figure 1 is a schematic sectional view of a medical injection device resulting from the present invention;
- Figure 2 is a graph showing the proportion of lubricant, measured by Atomic Absorption Spectrometry, leaching from the syringe barrel for a conventional (Prior-art) storing method and for a method according to the present invention (PTSi). Two emulsion-based adjuvants (Composition 1 and Composition 2) were investigated;

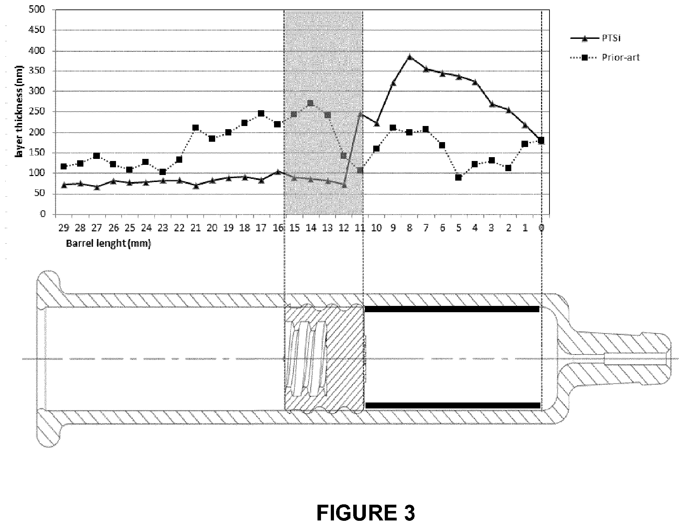

- Figure 3 is a graph showing the variation of the thickness of the lubricant coating measured by Silicone Layer Explorer when Composition 2 is stored according to the present invention (PTSi) and according to a conventional method (Prior art)

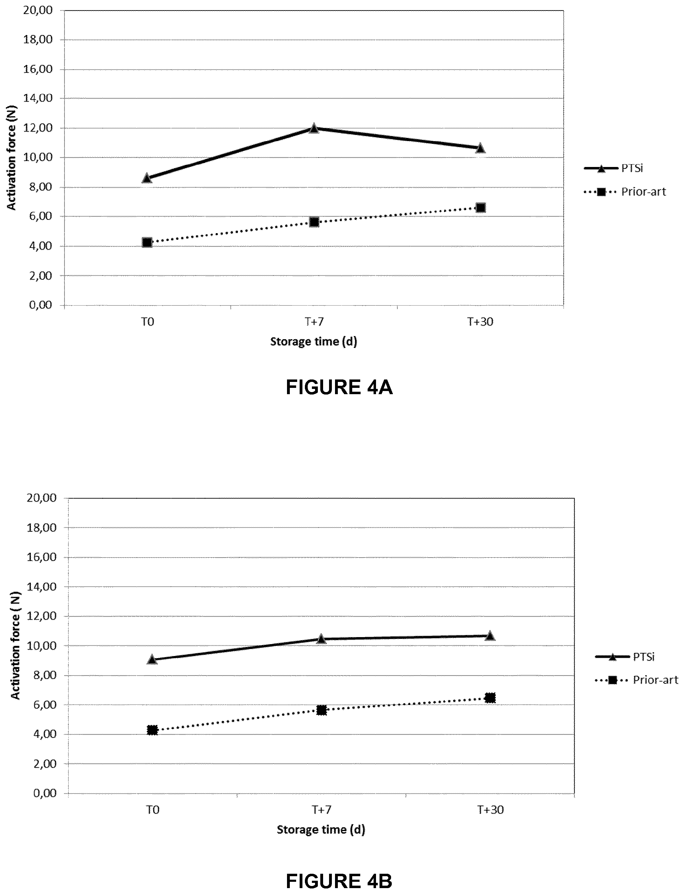

- Figures 4A and 4B show respectively the activation force of the stopper for each of the storing method when the syringe barrel is filled either with Composition 1 and Composition 2, the measurements being performed immediately after filling (T0), after 7 days of storage (T+7) and after 30 days of storage (T+30);

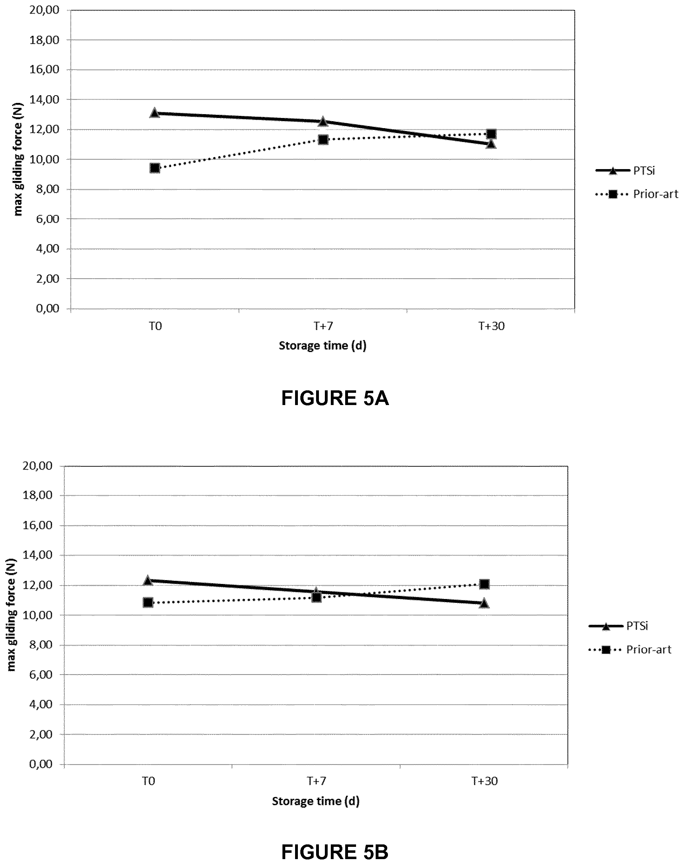

- Figures 5A and 5B show respectively the maximum gliding force of the stopper when respectively Composition 1 and Composition 2 are stored according to the prior-art method and according to the method of the present invention. The measurements have been performed immediately after filling (T0), after 7 days of storage (T+7) and after 30 days of storage (T+30).

### DETAILED DESCRIPTION OF THE INVENTION

### Medical injection device

Referring to Figure 1, the injection device 1 comprises a container, such as a barrel 2, and a stopper 3 in gliding engagement within the barrel 2.

The barrel 2 of the injection device may be made of any kind of glass or plastic suitable for medical applications.

The stopper 3 may be made of any elastomeric material, e.g. rubber, butyl rubber, silicone rubber, coated or not with an inert film.

The stopper 3 may be adapted to be connected, for example, to a plunger rod of a syringe or of an injection pump (not illustrated).

To that end, it includes any suitable connecting means, e.g. a threaded portion 31, etc.

The end 20 of the barrel 2 opposed to the stopper 3 may be adapted to be connected to a needle, a cap or a catheter, or may accommodate a staked needle.

The barrel is intended to contain an emulsion-adjuvanted vaccine 6 as described above. In other embodiments (not shown), the barrel may contain any pharmaceutical or diagnostic compositions containing an emulsion.

According to the storing method of the present invention, at least the part of the inner wall 21 of the barrel 2 that is in contact with the vaccine is coated with a lubricant coating 5, which results from an oxidizing plasma treatment of a silicone oil layer.

As will be explained in more details below, the storing method of the present invention allows a low level of interaction between an emulsion-based vaccine adjuvant and the medical container.

According to an embodiment of the invention, the formation of the coating 5 preferably comprises, in a first step, the formation of a silicone oil layer on the inner wall of the barrel.

The formation of said layer may be accomplished by any suitable technique, e.g. spraying, dipping, spin coating, etc. in order to obtain a layer thickness comprised between 90 and 400 nm, preferably about 150 nm, depending on the nature of the surface but also the material of medical injection device.

As will be described in more detail below, the layer thickness may be measured by reflectometry, for example by a rap.ID Layer Explorer.

According to an embodiment, the silicone oil essentially consists in a poly(alkylsiloxane) or a poly(dialkylsiloxane) and preferably the alkyl side chains comprise one to four carbon atoms.

According to other embodiments, the alkyl side chains may comprise reactive groups such as hydroxyl, formyl, keto, carbonyl or may include ether or ethyleneglycol.

In a preferred embodiment, the silicone oil essentially consists in poly(dimethylsiloxane) PDMS and in this preferred solution, the viscosity of the PDMS is comprised between 900 and 1200 centiStokes (cSt), at 25°C.

More preferably, the silicone oil is a 1000-centiStoke (at 25°C) PDMS, such as PDMS DC-360 from Dow Corning^{®}.

The silicone oil is applied so as to cover the major part of the interior wall 21 of the barrel.

Preferably, the silicone oil covers at least 90% of the interior surface of the barrel 2. For example, a quantity of 0.1 to 0.4 mg of silicone oil, more preferably 0.4 to 0.5 mg, may be used for a 1 ml standard glass syringe.

In a second step, the plasma treatment is applied in order to crosslink the silicone oil into a lubricant coating. The plasma treatment is preferably applied in an oxidizing atmosphere.

According to a preferred embodiment, the atmosphere is a mixture of 15 to 30% of oxygen and 85 to 70% of argon, in term of partial pressures. More preferably, the atmosphere comprises 25% of oxygen and 75% of argon.

Generally speaking, the plasma may be produced by different techniques such as corona discharge, microwave, radio-frequency or any other convenient methods.

Preferably, a radio frequency plasma treatment is used, i.e. with a frequency ranging from 10 to 20 MHz, more preferably from 11 to 14 MHz.

The power applied to generate the plasma may be comprised between 50 and 300 W, preferably between 100 and 250 W and the plasma treatment may be carried out at room temperature (i.e. 25°C) and under a vacuum ranging from 1.33 to 13.3 Pa (10 to 100 mTorr) in absolute value.

The exposure time of the silicone oil layer to the plasma is typically comprised between 10 to 40 seconds.

The above parameters depend on the plasma reactor geometry, the surface and volume of the medical device comprising the lubricant coating, the arrangement of the devices inside the plasma reactor, the thickness of the lubricant layer, etc.

As a consequence, tiny changes in parameters can lead to a different coating and the skilled person may select the parameters of the plasma treatment in order to optimize the treatment depending on the equipment used as well as on the devices to be treated.

As a result of the plasma treatment, the silicone oil is oxidized and crosslinked to a lubricant coating.

For a 1 ml standard syringe having a glass barrel, the preferred conditions of the plasma treatment are:
- plasma type: capacitively coupled plasma driven by a radiofrequency power supply at 13.56 MHz;
- treatment time: 30 s;
- power: 200 W;
- pressure: 8 Pa (0.06 Torr);
- atmosphere: 15/75 partial pressure of O₂ and Ar, respectively.

This treatment is applied to a syringe containing between 0.4 and 0.5 mg of silicone oil as a layer on the internal barrel surface, which corresponds to a thickness between 90 and 400 nm.

According to an advantageous embodiment of the invention, the stopper is also lubricated in order to further reduce the gliding force.

In a preferred example, the stopper is a rubber stopper covered by a fluoropolymer laminated film, such as the Flurotec^{®} stopper sold by West Pharmaceutical Services, and further siliconized by PDMS DC-360.

The plasma treatment applied to the stopper (if any) is the same as the treatment described above for the barrel.

According to an embodiment, the medical injection device may advantageously be used as a prefilled injection device, i.e. an injection device that is filled with a pharmaceutical composition before being sold or delivered to end-users. For example, the injection device can be a syringe filled with a pharmaceutical composition such as a vaccine. Particularly, the vaccines mixed with emulsion adjuvants are pharmaceutical compositions that can be stored in the medical injection devices according to the present invention. More precisely, the emulsion-adjuvanted vaccines can also be placed in a syringe intended to be filled and stored before use.

The storing method of the present invention allows the storage of the medical injection device in different conditions and positions, even during a long period, as well as transporting it over long distances, without affecting the integrity of the vaccine and the functionality of the lubricant coating, especially because there is a low level of interaction with the emulsion-adjuvanted vaccine.

### Vaccine formulation

An emulsion-adjuvanted vaccine comprises an active agent, e.g. an antigen, and an emulsion acting as an adjuvant of the active agent.

According to a preferred embodiment, said emulsion is an oil-in-water emulsion and preferably the oil phase can comprise squalene. Other oil phases could be chosen without departing from the scope of the invention, for example such oils can be selected from terpenes, terpenoides or derivatives thereof. Besides, the invention is not limited to a specific type of active agent and all drugs requiring an emulsion may be stored according to the present invention. An emulsifier, such as a surfactant, is added to the oil phase in order to stabilize the emulsion while the active agent of the vaccine is contained in the water phase of the emulsion. Surfactants may be chosen among all surfactants authorized for medical use, for example sorbitans, fatty acids, tocopherols, polysorbates, fatty alcohols and combinations and derivatives thereof.

In the present invention two different emulsions-based adjuvants have been studied. The composition of such emulsions-based adjuvant and the corresponding emulsification process are the following:
Composition 1: 4.3% w/v squalene, 4.75% w/v α-tocopherol, 1.94% w/v Polysorbate 80 in Phosphate Buffered Saline pH 6.8; micro-fluidization;
Composition 2: 3.3% w/v squalene, 0.57% w/v sorbitan monooleate , 0.72% w/v Ceteareth-12 in Phosphate Buffered Saline pH 7.4; phase-inversion temperature process;
wherein polysorbate 80 is Tween^{®} 80, sorbitan monoleate is Montane^{®}80, Ceteareth-12 is Emulgin^{®}B1 PH. Phosphate Buffered Saline is a mixture of NaCl, KCI, Na₂KHPO₄ and KH₂PO₄, set at the required pH.

Each of these compositions has been diluted with Phosphate Buffer Saline, with the same dilution rate as used when these compositions are mixed with a vaccine antigen. This dilution rate is 2 times (2x) for Composition 1 and 1.5 times (1.5x) for Composition 2.

### Experimental data

The storing method of the present invention has been compared with a prior-art storing method using two different emulsion formulations, namely Compositions 1 and 2, as described above.

For such experiments, a medical injection device obtained according to the method of the invention (hereinafter noted "PTSi") has been tested and compared with a prior-art medical injection device comprising a glass barrel and lubricated with pure DC-360 PDMS (1000 cSt), hereinafter noted "Prior-art". The PTSi syringes were 1 ml standard glass syringes siliconized with 0.4 mg of PDMS DC-360 and treated with a plasma process according to the present method. Half of these syringes were filled with Composition 1 and another half with Composition 2. All needles were closed with a standard rigid needle shield (RNS), the syringes were stopped with siliconized 3-rib rubber stoppers and stored 6 weeks at 25°C and 60 % humidity.

The Prior-art references were prepared using the same method but without the plasma treatment included in the present invention.

Experiments have been conducted to measure the silicone migration from the lubricant coating to the emulsion-based adjuvants, to evaluate the thickness of lubricant coating remaining onto the syringes internal surfaces and to measure the activation and gliding forces of the stoppers into the syringe barrels.

### Silicone migration

Silicone migration has been measured for both emulsion adjuvants, when stored in siliconized syringes according to the prior-art method and compared to a storage realized according to the present invention.

After the 6-week storage time, the syringes were opened and the liquid removed. The lubricant remaining on the internal surface of the syringe barrels was dissolved by adding MethyllsoButylKetone (MIBK) into the syringe barrels.

To ascertain that all the lubricant was dissolved, the syringes full of MIBK were placed into an ultrasonic bath VWR^{®} USC1200D during 15 min at 160W and 45 kHz, the bath temperature being 40 °C.

The silicone quantity dissolved in MIBK (in µg) was then measured by Atomic Absorption Spectroscopy (AAS) using an Atomic Analyst 800 from Perkin Elmer^{®} in Flame mode. The spectrometer was further provided with a hollow cathode silicone lamp used at a working wavelength of 251.6 nm with a 0.2H bandwidth. The lamp was provided with 35 mA power supply.

Before the analysis, the burner of the spectrometer was supplied with a mixture of air and acetylene (18.0 and 2.5 I/min respectively) for about 5 minutes for a proper warming up while air, water and ethanol were sequentially pumped in the analytical circuit.

The burner was then supplied with a mixture or nitrous oxide and acetylene (16.0 and 5.8 I/min respectively) during 5 minutes while ethanol have been pumped in the analytical circuit.

After such preparation of the equipment, a blank was realized with pure MIBK and a calibration curve was performed by successive dilution of a stock solution of 500 ppm PDMS in MIBK (50 mg PDMS DC-360, 1000 cSt, in 200 ml MIBK).

The quantity of silicone measured by this experiment is the quantity of silicone remaining into the lubricant coating after the storage time. The quantity of silicone that migrated in the emulsion is then obtained by difference with the initial quantity of silicone introduced in the syringe barrel. The relative quantity of silicone leaching into the emulsion is finally obtained by dividing the quantity of migrated silicone with the initial quantity of silicone, and the results are shown on Figure 2.

For both Composition 1 and Composition 2 the relative quantity of lubricant leaching from the barrel after 6-week storage is significantly reduced when the emulsion compositions are stored according to the present invention.

Indeed, more than 30 % of the lubricant amount is removed from the syringe barrel coated with non-crosslinked PDMS because of the dissolving action of the adjuvant composition, whereas only 10 % of the coating according to the present method was removed when stored with Composition 1 and only 17 % when stored with Composition 2.

As a result, more than 80 % of the lubricant is still present on the barrel surface for both Composition 1 and 2, which ensures an optimal gliding of the syringe stopper at the time of injection. It can then be concluded that a pharmaceutical composition comprising these emulsion-based adjuvants can be preserved during an extended storage time and safely injected to people.

### Silicone layer thickness

The thickness variation of the silicone coating along the barrel length has also been measured by reflectometry with a Layer Explorer from rap.ID Particle Systems.

In this experiment, six syringes were filled with Composition 2. After a 6-week storage time, the liquid of Composition 2 was removed from the syringe and the lubricant layer thickness was measured with the Layer Explorer apparatus. The refraction index was set to 1 and the layer thickness has been measured on eight different lines along the barrel length, each line being spaced from 45° as regards as the other lines. On each line, the layer thickness was measured on each millimeter of the barrel length. The arithmetic mean of the layer thickness was then calculated based on the measured values for the eight lines of each tested syringe.

Figure 3 shows the average thickness (in nm) of the lubricant when Composition 2 is stored according to the present invention (plain line, triangle marker) and according to the prior art method (dotted line, square marker).

The right portion of the graph, referred as 0 mm, corresponds to the distal portion of the barrel while the left position of the graph, referred as 29 mm, corresponds to the proximal part of the barrel.

The grey region corresponds to the position of the stopper into the syringe barrel.

This graph shows that a sufficient coating thickness i.e. above 200 nm is preserved after extensive storage with Composition 2 i.e. with a squalene-based adjuvant composition, when the composition is stored according to the present invention.

As a result, the method of the present invention will thus maintain an optimal lubrication between the stopper and the syringe barrel, even after an extensive storage time.

On the contrary, the silicone layer used as lubricant for the prior-art syringes is partially solubilized by the adjuvant composition and only an insufficient layer thickness remains on the internal surface of the barrel. Lubrication of the stopper into the syringe barrel is thus dramatically reduced and patients or medical staff may experience stick/slip phenomenon. In some cases, the stopper may even be blocked into the syringe barrel, therefore leading to a non-functional syringe. Ultimately, such consequences could cause injuries during people immunization and diminish immunization campaigns efficiency.

### Activation force

The activation force has been measured to evaluate the functionality of the injection device when emulsion-adjuvanted vaccines are stored according to the present invention by comparison with the prior-art method.

Indeed, the activation force is the force to be applied to the stopper to initiate its sliding movement within the barrel.

The stopper usually consists of rubber and its usual behavior is a tendency to stick on the barrel surface over time, leading to an increase of the activation force. This phenomenon can be explained by the pressure applied by the stopper onto the lubricated syringe barrel which tends to push the lubricant outside of the interface. The activation force is thus directly linked with the ability of the lubricant to remain at the interface between the stopper and the syringe barrel.

A great activation force may lead to a non-convenient medical injection device because the user needs to apply a greater effort to move the stopper. It may also lead to injuries at the time of injection, because of uncontrolled movements of the syringe needle into the person's body or accidental pricking of the medical staff.

For measuring this activation force, syringes have been filled with Composition 1 and Composition 2 according to the previously described protocol (PTSi) and the prior-art method (Prior-art). The filled syringes have been connected to a traction-compression bench (Llyod LRX Plus) to induce gliding of the stopper within the container. This test has been performed immediately after stoppering (T0), after 7 days (T+7) and after 30 days (T+30) of storage time at 25°C and 60 % humidity. Fifteen syringes have been used for each activation force measurement.

Figures 4A and 4B show the activation force obtained in the syringes when respectively Composition 1 and Composition 2 are stored according to the method of the invention (plain line, triangle markers) and according to the conventional method (dotted line and square markers). For both cases, the activation force obtained with PTSi syringes is approximately comprised between 9 and 12 N, while the activation force obtained with prior-art syringes remains between 4 and 6N.

The general tendency is that the activation force increases with time, whatever the coating and the emulsion formulation. However, the activation force remains at a reasonable level as compared to prior-art syringes.

In addition, the PTSi coating, while not providing the lowest activation force, keeps a substantially stable and predictable activation force over time, which is particularly valuable in the case of prefilled syringes which are usually stored during an extended period of time before injection. The syringes prepared according to the present invention hence show a correct activation force for prefilled syringes i.e. below 12 N.

### Gliding force

The gliding force is also a parameter defining functionality of the medical injection device.

Indeed, the gliding force is the force to be applied to the stopper to maintain its sliding movement within the barrel, i.e. after the stopper has been set in motion by an activation force. This gliding force strongly depends on the lubricant layer conditions and the maximum gliding force is maximum force to apply by a user to sustain the stopper gliding movement.

Similarly to the activation force, a high gliding force may lead to a non-convenient medical injection device because it requires from the user a greater effort to move the stopper within the barrel. Furthermore, uncontrolled movement of the needle into the body may also result from a high gliding force.

Figures 5A and 5B show the maximum gliding force of the stopper (in N) obtained for each of the tested syringes immediately after filling (T0), after 7 days of storage (T+7) and after 30 days of storage (T+30) for Composition 1 and Composition 2, respectively. The storage of the syringes was realized at 25°C with 60 % humidity.

When stored according to the invention (PTSi), both Composition 1 and Composition 2 (plain line, triangle markers) show a slight decrease of the maximum gliding force over time. Indeed, the maximum gliding force after 30 days of storage is lower than the maximum gliding force immediately after filling. This phenomenon may be explained by the stabilization over time of the coating

On the contrary, syringes filled according to the prior-art method with the same compositions show a slight increase over time of the maximum gliding force, this force being higher after 30 days of storage than immediately after filling. This can be explained by the extraction of the silicone oil from the lubricant layer to the emulsion adjuvant composition. This phenomenon is particularly detrimental in the case of prefilled syringes, which are stored over an extended period of time before being used, as the maximum gliding force could reach high and unpredictable value.

Consequently, a syringe filled according to the present method is valuable to maintain a smooth gliding of the stopper into the syringe barrel at the time of injection, in particular after an extended storage. While allowing a comfortable injection for the medical staff, such a smooth movement also allows avoiding injuries that may happen when a high amount of force is required to perform the injection.

### Results and Analysis

The inventors have identified a destabilization phenomenon of emulsion-adjuvanted vaccines which is avoided by the previously described storing method.

Without being bound by theory, the inventors believe that squalene interacts with the silicone used as a lubricant and thus generate migration of silicone into the emulsion-adjuvanted vaccine composition.

The solubilized silicone is believed to extract and replace the squalene into the emulsion composition because of its high affinity with the squalene and the surfactants used to stabilize the emulsion.

Consequently, the adjuvant-based emulsion, contaminated by the solubilized silicone, tends to convert into two dissociated phases in order to reduce the surface tension. Then, the emulsion loses its stability, which may affect the potency of the vaccine.

On the other hand, migration of silicone into the vaccine reduces the gliding properties of the lubricant coating, thereby requiring high forces to sustain movement of the stopper into the syringe barrel. In the case of prefilled syringes, this phenomenon occurs during the whole storage time and may result in non-functional or dangerous syringes
The inventors have found that a storage method according to the present invention that is based on an oxidizing plasma treatment of silicone oil is less subject to silicone migration generated by squalene emulsion-based adjuvants than known silicone coatings.

As a result, the emulsion-based adjuvant shows a high stability over time when stored according to the present invention and is not subjected to destabilizing interactions with the silicone lubricant. This is particularly valuable to maintain vaccine potency over time and to decrease the risk of unpredictable side effects among the persons receiving the vaccines.

Besides, since silicone extraction from the lubricant coating is avoided or at least limited, the coating keeps its integrity during a long period of time, for example from 12 to 24 months.

This is particularly important when the prefilled medical container is stored for a long time in different conditions and positions, transported over long distances and then finally used to inject a pharmaceutical composition to people. The injection can be realized in a smooth and comfortable way, while reducing the risk of injuries that results from a high gliding force or stick-slip effects.

Another advantage of a method according to the invention is that no further chemical species is introduced into the medical container, since the only chemical consists in a classic silicone oil, already used and authorized for such use. In particular, this diminishes the risk of unpredictable side effects.

### REFERENCES

[1] J Haensler, "Oil emulsions as vaccine adjuvants", in Novel Immunologic Adjuvants, Rino Rappuoli & Ennio De Gregorio, Future Medicine Ltd., October 2011

## Claims

1. Method for reducing destabilizing interactions between a lubricant coating and an emulsion of an emulsion-adjuvanted vaccine in a medical injection device comprising a lubricant coating, said method being **characterized in that** it comprises:
(a) providing a medical injection device (1) comprising a barrel (2) having an inner surface (21);
(b) forming a silicone oil layer on at least a part of the inner surface (21) of the barrel that is intended to be in contact with the vaccine,
(c) carrying out an oxidizing plasma treatment of said layer so as to crosslink at least part of the silicone oil layer and form a lubricant coating (5),
(d) filling the barrel with the emulsion-adjuvanted vaccine (6), wherein the emulsion is an oil-in-water emulsion comprising an oil phase including squalene and a water phase,
wherein the plasma-treated silicone oil reduces destabilization of the emulsion-adjuvanted vaccine by reducing solubilization of the silicone oil into squalene and reducing dissociation of oil and water phases of the emulsion by solubilized silicone oil.

2. Method according to claim 1, wherein in step (a) the barrel (2) is made of glass.

3. Method according to one of claims 1 to 2, wherein in step (b) the silicone oil has a viscosity between 9 and 12 cm²/s at 25°C.

4. Method according to one of claims 1 to 3, wherein the coating (5) formed in step (c) has a thickness between 90 and 400 nm, measured by reflectometry.

5. Method according to one of claims 1 to 4, wherein in step (a) the medical injection device (1) comprises a syringe having an internal volume of 1 ml.

6. Method according to claim 5, wherein the silicone oil layer has a weight between 0.1 and 0.4 mg.

7. Method according to one of claims 1 to 6, further comprising providing a stopper (3) in gliding engagement with the inner surface of the barrel (2), and forming a silicone oil layer on at least a part of said stopper (3) that is intended to be in contact with the vaccine (6) and exposing said silicone oil layer to an oxidizing plasma treatment so as to form a lubricant coating on said part of the stopper.

8. Method according to one of claims 1 to 7, wherein at least 90% of the inner surface of the barrel (2) is coated with said lubricant coating (5).

9. Method according to any of claims 1 to 8, wherein the oxidizing plasma treatment is carried out in an atmosphere comprising oxygen and argon.

10. Method according to claim 9, wherein in step (c) the atmosphere of said oxidizing plasma contains oxygen and argon with respective partial pressures comprised between 15 and 30% for oxygen and between 85 and 70% for argon.

11. Method according to any of claims 1 to 10, **characterized in that** the exposure of the silicone oil layer to the plasma is performed during between 10 and 40 seconds.

12. Method according to any of claims 1 to 11, wherein the oxidizing plasma is generated by radio-frequency, with a power ranging from 50 to 300W, and under a vacuum in the range 1.33-13.3 Pa in absolute value.

13. Medical injection device (1) comprising a lubricant coating (5) and containing an emulsion-adjuvanted vaccine (6) in contact with said lubricant coating (5), the vaccine including an oil-in-water emulsion comprising an oil phase including squalene and a water phase, **characterized in that** said lubricant coating (5) is directly obtained by steps (a) to (c) of the method according to one of claims 1 to 12 so as to reduce destabilizing interactions between said lubricant coating and the emulsion of the emulsion-adjuvanted vaccine.

14. Use of plasma-treated silicone oil as a lubricant coating (5) in a medical injection device (1) comprising a barrel (2), for preventing migration of silicone from said lubricant coating (5) to an emulsion-adjuvanted vaccine (6) including an oil-in-water emulsion comprising an oil phase including squalene and a water phase during storage of said vaccine in said medical injection device so as to reduce destabilizing interactions between said lubricant coating and the emulsion of the emulsion-adjuvanted vaccine.

## Patentansprüche

1. Verfahren zur Reduzierung destabilisierender Wechselwirkungen zwischen einer Gleitmittelbeschichtung und der Emulsion eines per Emulsion adjuvantierten Impfstoffs in einer eine Gleitmittelbeschichtung umfassenden medizinischen Injektionsvorrichtung, wobei das Verfahren **dadurch gekennzeichnet ist, dass** es umfasst:
(a) Bereitstellen einer medizinischen Injektionsvorrichtung (1), die einen Zylinder (2) mit einer Innenfläche (21) umfasst;
(b) Bilden einer Silikonölschicht auf mindestens einem Teil der Innenfläche (21) des Zylinders, der mit dem Impfstoff in Kontakt sein soll,
(c) Durchführen einer oxidierenden Plasmabehandlung der Schicht, um mindestens einen Teil der Silikonölschicht zu vernetzen und eine Gleitmittelbeschichtung (5) zu bilden,
(d) Befüllen des Fasses mit dem per Emulsion adjuvantierten Impfstoff (6), wobei die Emulsion eine Öl-in-Wasser-Emulsion ist, die eine Ölphase einschließlich Squalen und eine Wasserphase umfasst,
wobei das plasmabehandelte Silikonöl die Destabilisierung des per Emulsion adjuvantierten Impfstoffs reduziert, indem es die Löslichkeit des Silikonöls in Squalen reduziert und die Dissoziation von Öl- und Wasserphasen der Emulsion durch gelöstes Silikonöl reduziert.

2. Verfahren nach Anspruch 1, wobei in Schritt (a) der Zylinder (2) aus Glas besteht.

3. Verfahren nach einem der Ansprüche 1 bis 2, wobei in Schritt (b) das Silikonöl eine Viskosität zwischen 9 und 12 cm²/s bei 25 °C aufweist.

4. Verfahren nach einem der Ansprüche 1 bis 3, wobei die in Schritt (c) gebildete Beschichtung (5) gemessen durch Reflektometrie eine Dicke zwischen 90 und 400 nm aufweist.

5. Verfahren nach einem der Ansprüche 1 bis 4, wobei in Schritt (a) die medizinische Injektionsvorrichtung (1) eine Spritze mit einem Innenvolumen von 1 ml umfasst.

6. Verfahren nach Anspruch 5, wobei die Silikonölschicht ein Gewicht zwischen 0,1 und 0,4 mg aufweist.

7. Verfahren nach einem der Ansprüche 1 bis 6, ferner umfassend das Bereitstellen eines Stopfens (3) in Gleitkontakt mit der Innenfläche des Zylinders (2) und das Bilden einer Silikonölschicht auf mindestens einem Teil des Stopfens (3), der mit dem Impfstoff (6) in Kontakt sein soll, und das Aussetzen der Silikonölschicht einer oxidierenden Plasmabehandlung, um eine Gleitmittelbeschichtung auf dem Teil des Stopfens zu bilden.

8. Verfahren nach einem der Ansprüche 1 bis 7, wobei mindestens 90 % der Innenfläche des Zylinders (2) mit der Schmiermittelbeschichtung (5) beschichtet ist.

9. Verfahren nach einem der Ansprüche 1 bis 8, wobei die oxidierende Plasmabehandlung in einer Atmosphäre durchgeführt wird, die Sauerstoff und Argon umfasst.

10. Verfahren nach Anspruch 9, wobei in Schritt (c) die Atmosphäre des oxidierenden Plasmas Sauerstoff und Argon mit jeweiligen Partialdrücken zwischen 15 und 30 % für Sauerstoff und zwischen 85 und 70 % für Argon enthält.

11. Verfahren nach einem der Ansprüche 1 bis 10, **dadurch gekennzeichnet, dass** die Silikonölschicht zwischen 10 und 40 Sekunden lang dem Plasma ausgesetzt wird.

12. Verfahren nach einem der Ansprüche 1 bis 11, wobei das oxidierende Plasma durch Hochfrequenz mit einer Leistung von 50 bis 300 W und unter einem Vakuum im Bereich von 1,33 bis 13,3 Pa Absolutwert erzeugt wird.

13. Medizinische Injektionsvorrichtung (1), die eine Gleitmittelbeschichtung (5) umfasst und einen per Emulsion adjuvantierten Impfstoff (6) in Kontakt mit der Gleitmittelbeschichtung (5) enthält, wobei der Impfstoff eine Öl-in-Wasser-Emulsion umfasst, die eine Ölphase einschließlich Squalen und eine Wasserphase umfasst, **dadurch gekennzeichnet, dass** die Gleitmittelbeschichtung (5) direkt durch die Schritte (a) bis (c) des Verfahrens nach einem der Ansprüche 1 bis 12 erzielt wird, um destabilisierende Wechselwirkungen zwischen der Gleitmittelbeschichtung und der Emulsion des per Emulsion adjuvantierten Impfstoffs zu reduzieren.

14. Verwendung von plasmabehandeltem Silikonöl als Gleitmittelbeschichtung (5) in einer medizinischen Injektionsvorrichtung (1), die einen Zylinder (2) umfasst, zur Verhinderung der Migration von Silikon von der Gleitmittelbeschichtung (5) zu einem per Emulsion adjuvantierten Impfstoff (6) einschließlich einer Öl-in-Wasser-Emulsion, die eine Ölphase einschließlich Squalen und eine Wasserphase während der Lagerung des Impfstoffs in der medizinischen Injektionsvorrichtung umfasst, um destabilisierende Wechselwirkungen zwischen der Gleitmittelbeschichtung und der Emulsion des per Emulsion adjuvantierten Impfstoffs zu reduzieren.

## Revendications

1. Procédé pour réduire les interactions déstabilisantes entre un revêtement lubrifiant et une émulsion d'un vaccin à adjuvant émulsionné dans un dispositif médical d'injection comprenant un revêtement lubrifiant, ledit procédé étant **caractérisé en ce qu'**il comprend :
(a) fournir un dispositif médical d'injection (1) comprenant un cylindre (2) ayant une surface intérieure (21),
(b) former une couche d'huile de silicone sur au moins une partie de la surface intérieure (21) du cylindre qui est destinée à être en contact avec le vaccin,
(c) effectuer un traitement plasma oxydant de ladite couche de manière à réticuler au moins une partie de la couche d'huile de silicone et à former un revêtement lubrifiant (5),
(d) remplir le cylindre avec le vaccin à adjuvant émulsionné (6), l'émulsion étant une émulsion huile dans eau comprenant une phase huileuse incluant du squalène et une phase aqueuse,
dans lequel l'huile de silicone traitée au plasma réduit la déstabilisation du vaccin à adjuvant émulsionné en réduisant la solubilisation de l'huile de silicone en squalène et en réduisant la dissociation des phases huile et eau de l'émulsion par l'huile de silicone solubilisée.

2. Procédé selon la revendication 1, dans lequel, à l'étape (a), le cylindre (2) est en verre.

3. Procédé selon l'une des revendications 1 à 2, dans lequel, à l'étape (b), l'huile de silicone a une viscosité comprise entre 9 et 12 cm²/s à 25°C.

4. Procédé selon l'une des revendications 1 à 3, dans lequel le revêtement (5) formé à l'étape (c) a une épaisseur comprise entre 90 et 400 nm, mesurée par réflectométrie.

5. Procédé selon l'une des revendications 1 à 4, dans lequel, à l'étape (a), le dispositif médical d'injection (1) comprend une seringue ayant un volume interne de 1 ml.

6. Procédé selon la revendication 5, dans lequel la couche d'huile de silicone a un poids compris entre 0,1 et 0,4 mg.

7. Procédé selon l'une des revendications 1 à 6, comprenant en outre la fourniture d'un bouchon (3) en prise glissante avec la surface intérieure du cylindre (2), et la formation d'une couche d'huile de silicone sur au moins une partie dudit bouchon (3) qui est destinée à être en contact avec le vaccin (6) et l'exposition de ladite couche d'huile de silicone à un traitement plasma oxydant de manière à former un revêtement lubrifiant sur ladite partie du bouchon.

8. Procédé selon l'une des revendications 1 à 7, dans lequel au moins 90 % de la surface intérieure du cylindre (2) est recouverte dudit revêtement lubrifiant (5).

9. Procédé selon l'une des revendications 1 à 8, dans lequel le traitement par plasma oxydant est effectué dans une atmosphère comprenant de l'oxygène et de l'argon.

10. Méthode selon la revendication 9, dans laquelle, à l'étape (c), l'atmosphère dudit plasma oxydant contient de l'oxygène et de l'argon avec des pressions partielles respectives comprises entre 15 et 30 % pour l'oxygène et entre 85 et 70 % pour l'argon.

11. Procédé selon l'une des revendications 1 à 10, **caractérisé par le fait que** l'exposition de la couche d'huile de silicone au plasma est réalisée pendant une durée comprise entre 10 et 40 secondes.

12. Procédé selon l'une des revendications 1 à 11, dans lequel le plasma oxydant est généré par radiofréquence, avec une puissance comprise entre 50 et 300 W, et sous un vide compris entre 1,33 et 13,3 Pa en valeur absolue.

13. Dispositif médical d'injection (1) comprenant un revêtement lubrifiant (5) et contenant un vaccin à adjuvant émulsionné (6) en contact avec ledit revêtement lubrifiant (5), le vaccin comprenant une émulsion huile dans eau comprenant une phase huileuse incluant du squalène et une phase aqueuse, **caractérisé en ce que** ledit revêtement lubrifiant (5) est obtenu directement par les étapes (a) à (c) du procédé selon l'une des revendications 1 à 12 de manière à réduire les interactions déstabilisantes entre ledit revêtement lubrifiant et l'émulsion du vaccin à adjuvant émulsionné.

14. Utilisation d'une huile de silicone traitée au plasma comme revêtement lubrifiant (5) dans un dispositif médical d'injection (1) comprenant un cylindre (2), pour empêcher la migration du silicone dudit revêtement lubrifiant (5) vers un vaccin à adjuvant émulsionné (6) comprenant une émulsion huile dans eau comprenant une phase huileuse incluant du squalène et une phase aqueuse pendant le stockage dudit vaccin dans ledit dispositif médical d'injection de manière à réduire les interactions déstabilisantes entre ledit revêtement lubrifiant et l'émulsion du vaccin à adjuvant émulsionné.
